# EUROPEAN PATENT APPLICATION

(11) **EP 4 119 118 A1**
(43) Date of publication of application: **18.01.2023**
(21) Application number: 21768501.5
(22) Date of filing: 12.03.2021
(51) Int. Cl.: A61H 23/02, A61F 7/00

(54) **COSMETIC TREATMENT DEVICE**

(30) Priority: 13.03.2020 JP 2020066293
(71) Applicant: Amply Co., Ltd., Tokyo, 104-0061 (JP); Rhythm Co., Ltd., Osaka-Shi, Osaka, 553-0003 (JP); Izawa, Yoshihiro, Yachiyo-shi, Chiba, 276-0020 (JP)
(72) Inventor: IZAWA, Yoshihiro, Yachiyo-shi, Chiba 276-0020 (JP)
(74) Representative: Winter, Brandl - Partnerschaft mbB
(86) International application number: PCT/JP2021/010084
(87) International publication number: WO 2021/182615

(57) **Abstract**

A cosmetic treatment device includes a treatment surface that faces a human body, at least three head portions, and an oscillation portion. The head portions are arranged at a spacing with each other on the treatment surface, each head portion including an ultrasonic vibrator and being configured to vibrate together with the ultrasonic vibrator. The oscillation portion is configured to cause the ultrasonic vibrators to vibrate.

## Description

### Technical Field

The present invention relates to a cosmetic treatment device that uses ultrasonic vibrations.

### Background Art

Patent Literature 1 discloses a facial massager in which a contact element is provided on a head portion of a handle portion, and an ultrasonic vibrator is provided within the contact element. According to Patent Literature 1, the facial massager is used by a user holding the handle portion with the hand and bringing the contact element into contact with the facial skin. Due to ultrasonic vibrations of the contact element, the facial massager provides a stimulus to the skin and facilitates the permeation of a serum. The skin cells are thereby activated to condition the facial skin.

### Citation List

### Patent Literature

Patent Literature 1: Japanese Utility Model Registration No. 3200262

### Summary of Invention

### Technical Problem

In the facial massager disclosed in Patent Literature 1, ultrasonic vibrations are transmitted from the contact element provided at the center of the head portion to the human body. Because ultrasonic vibrations are mechanical vibrations, the vibrations from the ultrasonic vibrator concentrate on the center portion of the head portion on which the contact element is provided, and it is therefore not possible to effectively apply the vibrations to the human body using the entire head portion.

It is an object of the present invention to provide a cosmetic treatment device that can effectively transmit ultrasonic vibrations to the human body.

### Solution to Problem

A cosmetic treatment device according to one aspect of the present invention includes a treatment surface that faces a human body, at least three head portions, and an oscillation portion. The head portions are arranged at a spacing with each other on the treatment surface, each head portion including an ultrasonic vibrator and being configured to vibrate together with the ultrasonic vibrator. The oscillation portion is configured to cause the ultrasonic vibrators to vibrate.

With the cosmetic treatment device described above, it is possible to cause an interference to occur between ultrasonic vibrations output from the head portions. As a result, the concentration of the intensities of the ultrasonic vibrations onto one location on the treatment surface is suppressed, and thus ultrasonic vibrations can be generated over a wide area of the treatment surface. As a result, it is possible to effectively transmit the ultrasonic vibrations to the human body.

In the cosmetic treatment device described above, the at least three head portions may be arranged to form a polygonal shape on the treatment surface.

With the cosmetic treatment device described above, it is possible to more effectively cause the ultrasonic vibrations generated from the head portions to interfere with each other, and transmit ultrasonic vibrations with a small intensity variation to the human body.

The cosmetic treatment device described above may include four head portions.

In the cosmetic treatment device described above, the oscillation portion may be configured to apply an electric signal of 20 kHz to 3 MHz to the ultrasonic vibrators.

In the cosmetic treatment device described above, each of the at least three head portions may further include an electrode portion that is brought into contact with the human body. The cosmetic treatment device described above may further include a voltage applying portion configured to apply a predetermined voltage to the electrode portions.

With the cosmetic treatment device described above, the head portions for transmitting ultrasonic vibrations to the human body can be also used as electrode portions for providing a different electric cosmetic treatment. It is thereby possible to provide a multifunctional cosmetic treatment device with a small number of constituent elements.

### Advantageous Effects of Invention

According to the aspects of the present invention described above, it is possible to effectively transmit ultrasonic vibrations to the human body.

### Brief Description of Drawings

FIG. 1 is a diagram showing an overall configuration of a cosmetic treatment device according to an embodiment of the present invention;
FIG. 2 is a block diagram showing an electric configuration of the cosmetic treatment device;
FIG. 3 is a cross-sectional view of head portions;
FIG. 4A is a diagram illustrating how ultrasonic vibrations are transmitted;
FIG. 4B is a diagram illustrating how ultrasonic vibrations are transmitted;
FIG. 5 is a flowchart illustrating an example of an operation performed by the cosmetic treatment device;
FIG. 6 is a diagram showing an overall configuration of a cosmetic treatment device according to a variation;
FIG. 7Ais a diagram showing an arrangement of head portions according to the variation;
FIG. 7B is a diagram showing an arrangement of head portions according to another variation; and
FIG. 7C is a diagram showing an arrangement of head portions according to yet another variation.

### Description of Embodiments

Hereinafter, a cosmetic treatment device according to an embodiment of the present invention will be described with reference to the drawings.

### 1. Overall Configuration of Cosmetic Treatment Device

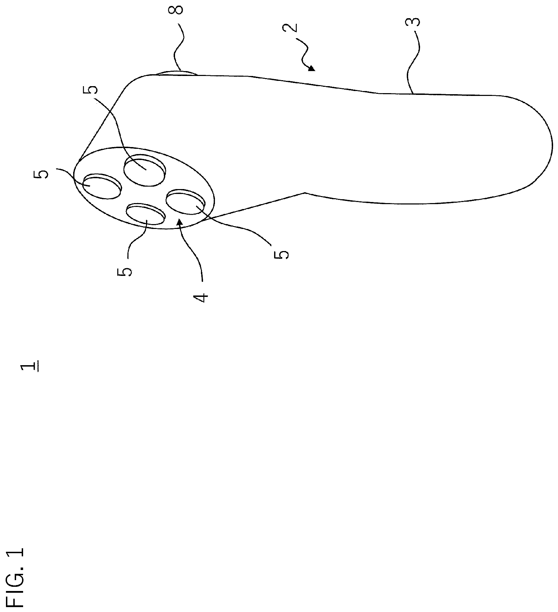

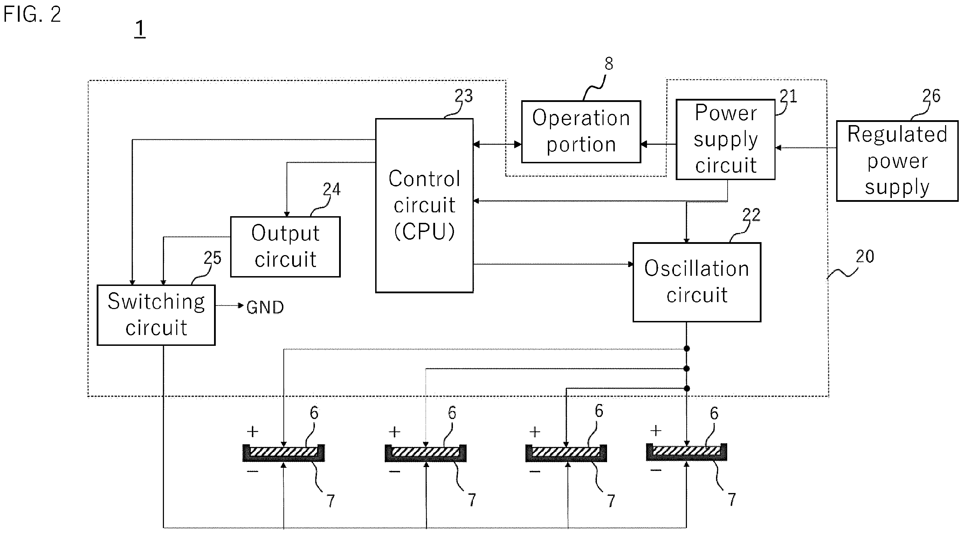

FIG. 1 is a diagram showing an overall configuration of a cosmetic treatment device 1 according to an embodiment of the present invention, and FIG. 2 is a block diagram showing an electric configuration of the cosmetic treatment device 1. The cosmetic treatment device 1 is configured to provide a cosmetic treatment by being connected to a power supply (not shown) and turned on to activate. The cosmetic treatment device 1 includes a casing 2 that forms a treatment surface 4 and a handle portion 3. A user who uses the cosmetic treatment device 1 holds the handle portion 3 such that the treatment surface 4 faces the human body, brings four head portions 5 arranged on the treatment surface 4 into contact with the skin, and activates the cosmetic treatment device 1. In this way, a cosmetic treatment can be provided to the human body. The skin may be the facial skin or the skin of a body part other than the face. As used herein, the user of the cosmetic treatment device 1 is typically a person who provides a cosmetic treatment to herself/himself using the cosmetic treatment device 1, but may be a person, such as, for example, an esthetician, who provides a cosmetic treatment to somebody else using the cosmetic treatment device 1.

The cosmetic treatment device 1 according to the present embodiment is configured to provide a treatment that uses ultrasonic vibrations (cavitation) and a treatment that uses electric signals. The treatment that uses electric signals may include, for example, an RF treatment that uses radio frequency (RF) waves and an EMS treatment that uses electrical muscle stimulation (EMS). The ultrasonic vibrations and the electric signals used for the treatment are output from four head portion 5. In other words, the head portions 5 function as heads for providing cavitation and also function as electrodes for providing the treatment that uses electric signals.

The casing 2 is made of, but not limited to, any of rigid resins, metals, and combinations thereof. An operation portion 8 is provided on the surface of the casing 2. By operating the operation portion 8, the user can turn on and off the cosmetic treatment device 1, select the type of treatment to be provided by the cosmetic treatment device 1, and adjust the intensity of ultrasonic vibrations output from the head portions 5. There is no particular limitation on the configuration of the operation portion 8 as long as it is possible to receive user operations. The operation portion 8 may have any configuration such as a push button, a switch lever, a rotatable dial, a touch panel display, or any of combinations thereof.

A control unit 20 that controls the operation of the cosmetic treatment device 1 is housed in the casing 2. The control unit 20 is electrically connected to the head portions 5 and the operation portion 8, and can control the operation of the cosmetic treatment device 1 in accordance with an operation performed on the operation portion 8. The control unit 20 includes a power supply circuit 21 that is connected to an external power supply (not shown) via a regulated power supply 26, an oscillation circuit 22, a control circuit 23, an output circuit 24, and a switching circuit 25. The regulated power supply 26 converts AC voltage to DC voltage. The operations of the circuits 21 to 25 will be described later.

### 2. Configurations of Constituent Elements

Hereinafter, the configurations of the constituent elements of the cosmetic treatment device 1 of the present embodiment will be described with reference to the drawings.

### Head Portion

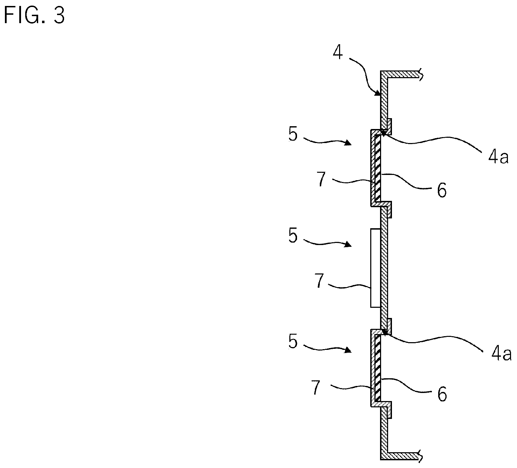

FIG. 3 is a cross-sectional view of the casing 2 showing the vicinity of the treatment surface 4. In the present embodiment, the treatment surface 4 is formed in a circular shape when viewed in a plan view. Four circular openings 4a are formed in a portion of the casing 2 that forms the treatment surface 4. The four openings 4a are formed at a predetermined spacing with each other on a concentric circle of the treatment surface 4. The head portions 5 are fixed to the inside of the casing 2 so as to be partially exposed on the treatment surface 4 via the openings 4a. That is, the head portions 5 are provided on the treatment surface 4 such that the head portions 5 are arranged at a predetermined spacing with each other. Accordingly, the head portions 5 exposed on the treatment surface 4 form a polygonal shape when the centers of the head portions 5 are connected. In the present embodiment, the head portions 5 are arranged to form a square shape.

### Electrode Portion

The head portions 5 each include an electrode portion 7. The electrode portion 7 is an electrode for applying electricity to the human body by outputting an electric signal while the head portion 5 is in contact with the skin surface, and is fixed to the inside of the casing 2 such that a portion of the electrode portion 7 is partially exposed on the treatment surface 4 via the opening 4a. That is, in FIG. 1, an exposed portion that is exposed on the treatment surface 4 as the head portion 5 is a portion of the electrode portion 7. Hereinafter, an exposed surface of the electrode portion 7 that is exposed on the treatment surface 4 will be referred to as "the surface of the electrode portion 7" and a surface that is opposite to the exposed surface will be referred to as "the back surface of the electrode portion 7". The electrode portions 7 are each made of a conductor, typically a metal such as stainless steel, aluminum, or an alloy. Furthermore, the surface of each electrode portion 7 may be plated with chromium as appropriate. The electrode portions 7 are electrically connected to the output circuit 24 via lead wires (not shown). As will be described later, a voltage of a predetermined frequency is applied to the electrode portions 7 by the output circuit 24. As a result, electric current flows from one electrode portion 7 to another electrode portion 7 that is paired therewith, mainly via the subcutaneous tissue.

There is no particular limitation on the frequency of the voltage applied to the electrode portions 7. However, in the case where the electrode portions 7 perform the RF treatment, the frequency may be set to, for example, 300 kHz to 3 MHz. In the case where the electrode portions 7 perform the EMS treatment, the frequency may be set to, for example, 20 Hz to 20 kHz.

### Ultrasonic Vibrator

The head portions 5 each further includes an ultrasonic vibrator 6. The ultrasonic vibrator 6 is fixed to the back surface of the electrode portion 7 using a chemical method or a mechanical method. Accordingly, the ultrasonic vibrators 6 are provided at a predetermined spacing with each other to form a square shape on the concentric circle, as with the electrode portions 7. The ultrasonic vibrators 6 are each made of a piezoelectric body that contracts upon application of a voltage and generates a voltage upon application of an external force, the piezoelectric body being typically made of a piezoelectric ceramic. The ultrasonic vibrators 6 are electrically connected to the oscillation circuit 22 via lead wires (not shown), and the ultrasonic vibrators 6 vibrate at a predetermined cycle when a voltage of a predetermined frequency is applied by the oscillation circuit 22. In the present embodiment, the ultrasonic vibrators 6 generate ultrasonic vibrations that are vibrations in the ultrasonic frequency band when a voltage of 20 KHz or more is applied by the oscillation circuit 22. The electrode portions 7 vibrate together with the ultrasonic vibrators 6, and transmit the ultrasonic vibrations to the skin that is in contact with the surface of the electrode portions 7.

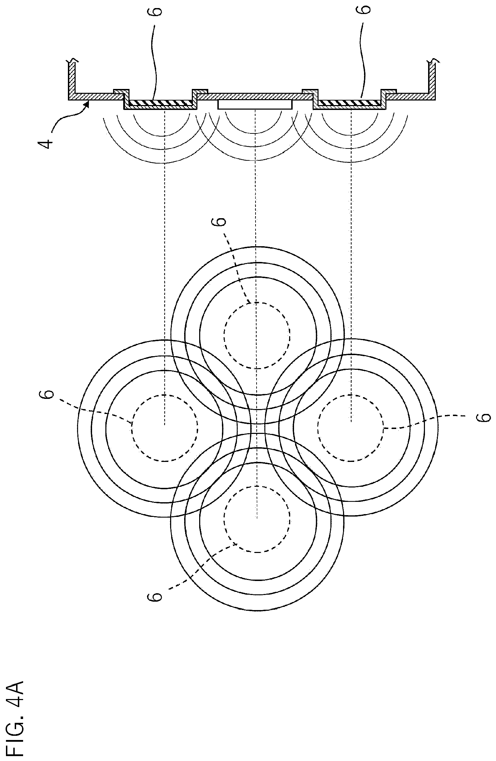

Voltage is applied to the ultrasonic vibrators 6 of the present embodiment by the same oscillation circuit 22. That is, four ultrasonic vibrators 6 generate ultrasonic vibrations in synchronization with each other. With this configuration, as shown in FIG. 4A, an interference between ultrasonic vibrations generated from the ultrasonic vibrators 6 occurs in the skin and the subcutaneous tissue, and it is therefore possible to output ultrasonic vibrations with a small intensity variation over a wide area of the treatment surface 4. Also, ultrasonic vibrations can be effectively transmitted to the skin surface and the subcutaneous tissue while controlling the intensity of ultrasonic vibrations generated by the individual ultrasonic vibrators 6 to a level that does not cause damage to the skin surface. There is no particular limitation on the frequency of the voltage applied to the ultrasonic vibrator 6. However, the frequency may be set to, for example, preferably 20 kHz to 3 Mhz, and more preferably 20 kHz to 100 kHz. In the present embodiment, the frequency is set to 40 kHz.

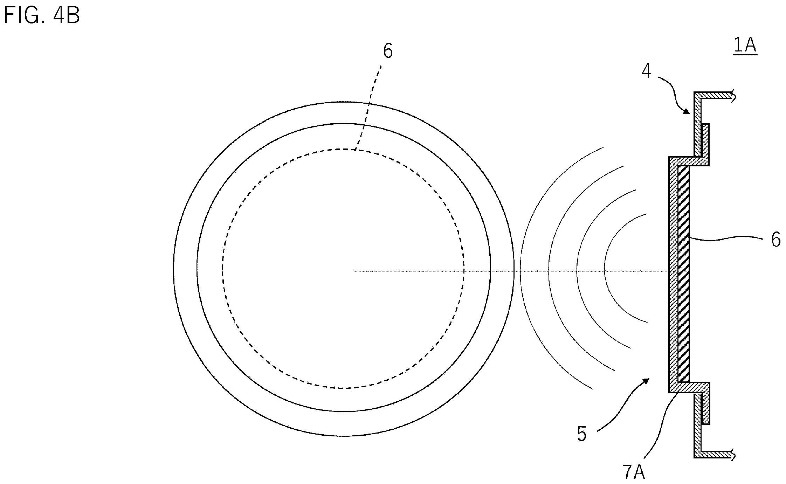

FIG. 4B is a diagram illustrating the cavitation performed by a cosmetic treatment device 1A that includes one head portion 5. In the cosmetic treatment device 1A, the head portion 5 is provided at the center of the treatment surface 4. The head portion 5 includes: a contact portion 7A that has a contact surface that is exposed on the treatment surface 4 and is brought into contact with the skin; and an ultrasonic vibrator 6 that is fixed to a surface of the contact portion 7A that is opposite to the contact surface. In the cosmetic treatment device 1A, the contact portion 7A vibrates together with the ultrasonic vibrator 6, and ultrasonic vibrations concentrate on one location on the treatment surface 4. Accordingly, the ultrasonic vibrations concentrate on a specific portion on the skin surface such as that occurs in a drum, as a result of which, a large variation occurs in the intensity of the ultrasonic vibrations as the entire treatment surface 4, and thus, even in a portion of the skin that faces the treatment surface 4, the ultrasonic vibrations may not be sufficiently transmitted. Also, due to attenuation of the ultrasonic vibrations, ultrasonic vibrations of a sufficient intensity are not transmitted to the subcutaneous tissue, and an efficient transmission of ultrasonic vibrations is no longer achieved. In contrast, when the intensity of ultrasonic vibrations that are output is increased, ultrasonic vibrations with a large amplitude may concentrate on a portion of the skin, which may cause damage to the skin. With the cosmetic treatment device 1 according to the present embodiment, the occurrence of situations as described above is suppressed.

### Control Unit

The control unit 20 is housed in the casing 2. The circuits 21 to 25 included in the control unit 20 can be mounted on a circuit board (not shown). The power supply circuit 21 receives a supply of electric power from an external power supply (not shown) via the regulated power supply 26, and drives the constituent elements of the control unit 20 with the electric power. In the present embodiment, the power supply circuit 21 is electrically connected to the operation portion 8, the oscillation circuit 22, and the control circuit 23.

The control circuit 23 controls the operations of the oscillation circuit 22, the output circuit 24, and the switching circuit 25. The control circuit 23 according to the present embodiment includes a CPU (Central Processing Unit), a nonvolatile rewritable storage device, a RAM (Random Access Memory), and a ROM (Read Only Memory). In the storage device or the ROM, a program for controlling the operations of the oscillation circuit 22, the output circuit 24, and the switching circuit 25 is stored. The CPU reads the program from the storage device or the ROM, and executes the program. The RAM is used by the CPU to perform calculation as appropriate.

With user operations via the operation portion 8, information for controlling operations is incorporated into the program. Information for controlling the durations of the treatment that includes the cavitation, the RF treatment, and the EMS treatment, the frequency and intensity of ultrasonic vibrations that are output, the frequency and intensity of electric signals that are output, and the like is incorporated into the program. The program performs control to continuously output ultrasonic vibrations or electric signals for a predetermined length of time or control to output ultrasonic vibrations or electric signals at a predetermined cycle repeatedly for a predetermined length of time or a predetermined number of times according to the type of treatment to be performed. Parameters such as output duration, cycle pattern, the number of cycles repeated, and the duration in which the repetition is performed can be pre-set. Hereinafter, a period in which a predetermined treatment is automatically performed in accordance with the pre-set parameters will be referred to as "a mode of the cosmetic treatment device 1".

In the present embodiment, the cosmetic treatment device 1 performs three modes including a cavitation mode, an RF treatment mode, and an EMS treatment mode. As will be described later, the cosmetic treatment device 1 is configured to perform these modes in a predetermined order in accordance with user operations. However, the cosmetic treatment device 1 may have the function of performing these modes alone in accordance with user operations received via the operation portion 8. Also, the cosmetic treatment device 1 may be configured to, even when any of these modes is being performed, perform operations such as receiving a user operation via the operation portion 8 as needed, adjusting the intensity of ultrasonic vibrations that are output, and changing the mode performed.

The oscillation circuit 22 is configured to be electrically connected to four ultrasonic vibrators 6, and apply a voltage of a predetermined frequency to each of the ultrasonic vibrators 6. That is, the oscillation circuit 22 corresponds to the oscillation portion of the present invention. With the oscillation circuit 22, the ultrasonic vibrators 6 ultrasonically vibrate at a predetermined frequency together with the electrode portions 7, and ultrasonic vibrations are transmitted to the human body. The magnitude of the voltage applied by the oscillation circuit 22, the frequency of the voltage applied by the oscillation circuit 22, and the duration in which the voltage is applied are controlled by the control circuit 23.

The output circuit 24 is configured to be electrically connected to four electrode portions 7, and apply a voltage of a predetermined frequency to each pair of electrode portions 7. That is, the output circuit 24 corresponds to the voltage applying portion of the present invention. With the output circuit 24, an electric signal with a predetermined waveform is output from the electrode portions 7 to the human body. The magnitude of the voltage applied by the output circuit 24, the frequency of the voltage applied by the output circuit 24, and the duration in which the voltage is applied are controlled by the control circuit 23.

The switching circuit 25 is a circuit configured to switch the type of treatment performed by the head portions 5, and may be implemented as appropriate using a relay circuit or the like. The switching circuit 25 switches the circuits to perform either one of the cavitation or the treatment that uses electric signals in accordance with a user operation or the mode performed by the program described above.

### 3. Operation of Cosmetic Treatment Device

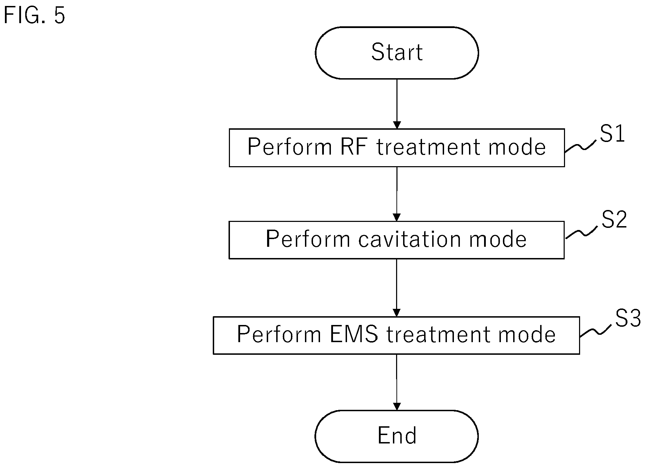

Hereinafter, an example of an operation of the cosmetic treatment device 1 according to the present embodiment will be described. FIG. 5 is a flowchart illustrating processing of performing the modes of the cosmetic treatment device 1. The processing shown in FIG. 5 starts when the user turns on the cosmetic treatment device 1 and inputs an operation to start a treatment via the operation portion 8, and ends when a series of operations have been performed. While steps S1 to S3 are being performed, the user brings the head portions 5 into contact with the skin by holding the handle portion 3, and moves the cosmetic treatment device 1 as appropriate such that the head portion 5 moves on the skin. It is preferable that a skin lotion, a serum, a gel cosmetic, or the like is applied as appropriate to the skin surface on which the treatment is performed.

In step S1, the RF treatment mode is performed. In the RF treatment mode, the control circuit 23 causes the electrode portions 7 to perform the RF treatment via the output circuit 24. That is, the output circuit 24 applies a voltage in the radio frequency band to the electrode portions 7, and then, an electric signal is transmitted from the electrode portions 7 to the subcutaneous tissue. There is no particular limitation on the frequency of the electric signal, but the frequency of the electric signal may be set to, for example, 300 kHz to 3 MHz. For example, the frequency of the electric signal may be set to 800 kHz. With the RF treatment, the water molecules in the subcutaneous tissue vibrate and generate heat, and thus improvement in the blood flow and the metabolic function can be achieved.

In the RF treatment mode, the RF treatment is performed continuously for a predetermined length of time or repeatedly in a predetermined cycle with a periodic temporary interruption. Also, in the RF treatment mode, the electrode portions 7 that apply voltage may be changed as appropriate to change the area where electricity is applied. The RF treatment mode ends when a predetermined length of time passes after the start of step S1, and the mode is switched to the cavitation mode. That is, the processing advances to step S2.

In step S2, the cavitation mode is performed. In the cavitation mode, the control circuit 23 causes four ultrasonic vibrators 6 to vibrate in synchronization with each other via the oscillation circuit 22, and causes four head portions 5 to perform cavitation. In the cavitation, not only ultrasonic vibrations generated from the head portions 5 are transmitted directly to the subcutaneous tissue via the skin, but also interference waves of ultrasonic vibrations as described above are generated in the subcutaneous tissue. It is thereby possible to suppress variation in the intensity of ultrasonic vibrations in an area on the skin that faces the treatment surface 4 and effectively cause ultrasonic vibrations to act on the subcutaneous tissue. Also, the subcutaneous tissue warmed by the RF treatment is further warmed, and thus the metabolic function is further improved.

In the cavitation mode, the cavitation is performed by the head portions 5 continuously for a predetermined length of time or repeatedly with a periodic temporary interruption. The cavitation mode ends when a predetermined length of time passes after the start of step S2, and the mode is switched to the EMS treatment mode. That is, the processing advances to step S3.

In step S3, the EMS treatment mode is performed. In the EMS treatment mode, the control circuit 23 causes the electrode portions 7 to perform the EMS treatment via the output circuit 24. That is, the output circuit 24 applies a voltage of 20 Hz to 20 kHz to the electrode portions 7, and an electric signal is transmitted from the electrode portions 7 to the subcutaneous tissue including muscles, capillaries, and lymphatic ducts. As a result, the muscles contract, and thus the muscle strength as well as skin sags and wrinkles are improved. Also, the flow in the capillaries and the lymphatic ducts is improved, which facilitates the discharge of waste and a further improvement of metabolism.

In the EMS treatment mode, the EMS treatment is repeatedly performed by the head portions 5 continuously for a predetermined length of time or repeatedly with a periodic temporary interruption. Also, in the EMS treatment mode, the electrode portions 7 that apply voltage may be changed as appropriate to create a bodily sensation as if the head portions 5 stimulate the subcutaneous tissue while the head portions 5 are moving. The EMS treatment mode ends when a predetermined length of time passes after the start of step S3, and the processing of performing the series of operation modes by the cosmetic treatment device 1 ends.

The processing of performing the modes described above is merely an example. Accordingly, the order in which steps S1 to S3 are performed may be changed as appropriate. Also, steps S1 to S3 may be repeated a predetermined number of times. Furthermore, a standby mode in which no treatment is performed may be provided during a period in which a transition is made from one mode to another mode. The standby mode may be set such that the next mode starts after the standby mode has been continued for a predetermined length of time, or may be set such that the next mode starts when an operation to execute the next mode is input by the user during the standby mode.

### 4. Variations

An embodiment of the present invention has been described above, but the present invention is not limited to the embodiment given above, and various modifications may be made without departing from the scope of the present invention. For example, modifications as described below can be made. Also, the gist of variations given below may be combined as appropriate.

### Variation 4-1

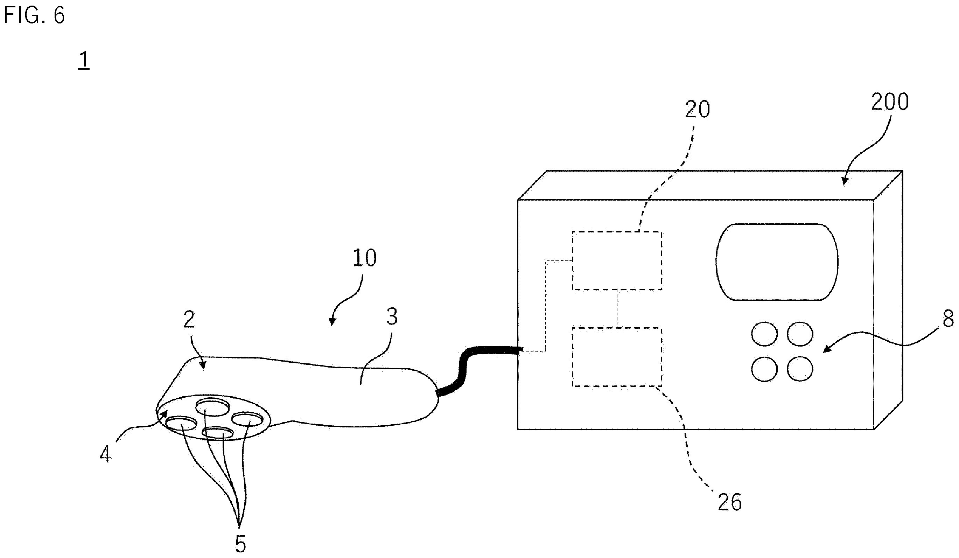

In the embodiment given above, the control unit 20 of the cosmetic treatment device 1 is housed in the casing 2. However, the cosmetic treatment device 1 may be configured as a device as shown in FIG. 6 that includes a probe 10 and a control box 200 that houses the control unit 20. In the cosmetic treatment device 1, the probe 10 includes: a casing 2 that forms a handle portion 3 and a treatment surface 4; and head portions 5. The head portions 5 are electrically connected to the control unit 20 of the control box 200. In this case, an operation portion 8 may be provided on the surface of the control box 200. Also, in this case, a regulated power supply 26 may be housed in the control box 200.

### Variation 4-2

In the embodiment given above, the cosmetic treatment device 1 is configured to be driven by an external power supply. However, in addition to or instead of this configuration, the cosmetic treatment device 1 may be configured to be driven by a battery.

### Variation 4-3

In the embodiment given above, the cosmetic treatment device 1 is configured to perform the cavitation and the treatment that uses electric signals, but the cosmetic treatment device 1 may be configured to perform only the cavitation, and either one of the RF treatment or the EMS treatment may be omitted. Also, the cosmetic treatment device 1 may further include, in addition to the head portions 5 to which the ultrasonic vibrators 6 are fixed, electrode portions 7 to which no ultrasonic vibrators 6 are fixed, and at least one of the RF treatment and the EMS treatment may be performed using the electrode portions 7 to which no ultrasonic vibrators 6 are fixed.

### Variation 4-4

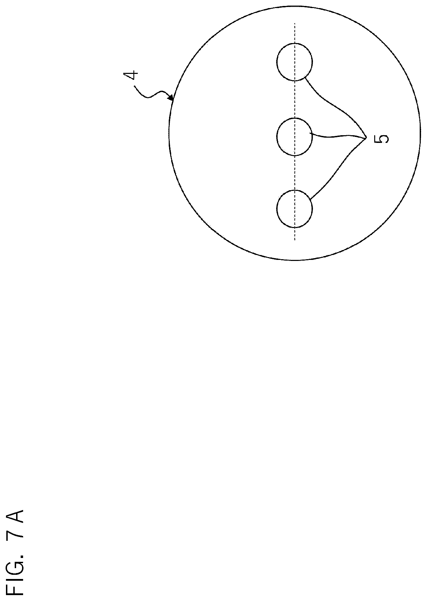

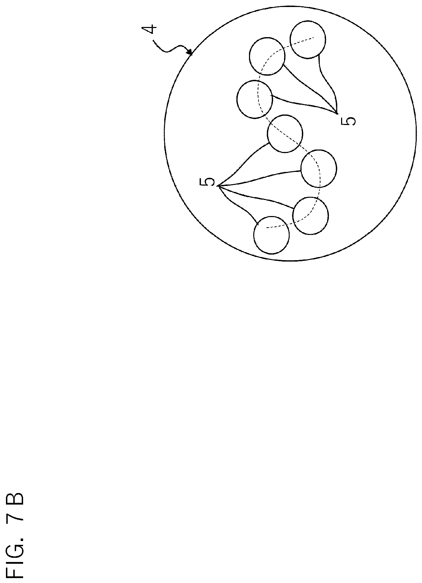

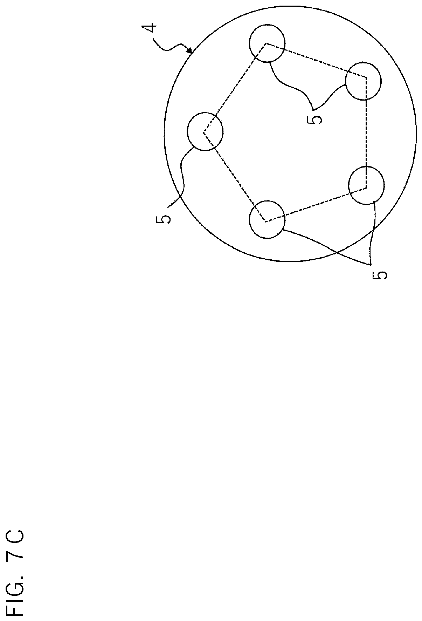

The number of head portions 5 provided on the treatment surface 4 is not limited to that mentioned in the embodiment given above, and may be 3, or 5 or more. As a result of at least three head portions 5 being provided at a predetermined spacing on the treatment surface 4, it is possible to effectively cause individual ultrasonic vibrations to interfere with each other. Also, there is no particular limitation on the shape and the arrangement of the head portions 5. The head portions 5 may be provided, for example, in a line as shown in FIG. 7A, in a curve as shown in FIG. 7B, or in a closed shape as shown in FIG. 7C. From the viewpoint of effectively causing ultrasonic vibrations to interfere with each other, it is preferable that at least three head portions 5 are arranged to form a polygonal shape on the treatment surface 4. Furthermore, the shape of the treatment surface 4 when viewed in a plan view is not limited to a circular shape, and may be changed as appropriate to, for example, a rectangular shape, a polygonal shape other than the rectangular shape, an elliptic shape, or the like, and may be combined with the number of head portions 5, the arrangement of the head portions 5, and the shape of the head portions 5.

### List of Reference Numerals

- 1: Cosmetic treatment device
- 2: Casing
- 3: Handle portion
- 4: Treatment surface
- 5: Head portion
- 6: Ultrasonic vibrator
- 7: Electrode portion
- 8: Operation portion
- 22: Oscillation circuit (oscillation portion)
- 24: Output circuit (voltage applying portion)

## Claims

1. A cosmetic treatment device comprising:
a treatment surface that faces a human body;
at least three head portions that are arranged at a spacing with each other on the treatment surface, each head portion including an ultrasonic vibrator and being configured to vibrate together with the ultrasonic vibrator; and
an oscillation portion that causes the ultrasonic vibrators to vibrate.

2. The cosmetic treatment device according to claim 1,
wherein the at least three head portions are arranged to form a polygonal shape on the treatment surface.

3. The cosmetic treatment device according to claim 1 or 2, comprising four head portions, each head portion including an ultrasonic vibrator and being configured to vibrate together with the ultrasonic vibrator.

4. The cosmetic treatment device according to any one of claims 1 to 3,
wherein the oscillation portion is configured to apply an electric signal of 20 kHz to 3 MHz to the ultrasonic vibrators.

5. The cosmetic treatment device according to any one of claims 1 to 4,
wherein each of the at least three head portions further includes an electrode portion that is brought into contact with the human body, and
the cosmetic treatment device further includes a voltage applying portion configured to apply a predetermined voltage to the electrode portions.
